Europäisches Patentamt

(19) European Patent Office

Office européen des brevets

(11) Publication number: **0 254 081**
**A2**

(12) **EUROPEAN PATENT APPLICATION**

(21) Application number: **87109352.2**

(22) Date of filing: **29.06.87**

(51) Int. Cl.³: **G 01 N 33/68**
**G 01 N 33/76, G 01 N 33/577**

(30) Priority: **30.06.86 JP 153707/86**

(43) Date of publication of application:
**27.01.88 Bulletin 88/4**

(84) Designated Contracting States:
**DE FR GB NL**

(71) Applicant: **Yoneda, Yuko**
**1-16 Akebono-cho**
**Toyama-shi Toyama-ken(JP)**

(72) Inventor: **Yoneda, Yuko**
**1-16 Akebono-cho**
**Toyama-shi Toyama-ken(JP)**

(74) Representative: **Patentanwälte TER MEER - MÜLLER -**
**STEINMEISTER**
**Mauerkircherstrasse 45**
**D-8000 München 80(DE)**

(54) **Method of detecting specific protein.**

(57) An invention for detecting specific protein (or peptide) has been developed with the following features:

1. Through a combination of monoclonal antibodies and/ or polyclonal antibodies, both of which have same specificity, proteins (or peptides) can be recognized and detected by using with an immunological reaction.

2. One of the detecting agent is a test piece of paper to which one monoclonal antibody or more, or one polyclonal antibody are fixed. A small amount of the test sample is placed on the paper to which the detecting solution (metallic colloidal solution bonded with a monoclonal antibody) is successively added. In case of a positive reaction a unique colored spot immediately becomes visible.

3. A filtration apparatus was developed for pretreatment of the test sample to remove the obstructive residue which disturbs the strict detection.

4. The invention includes the necessary equipment to make the detection.

EP 0 254 081 A2

0254081

# TER MEER - MÜLLER - STEINMEISTER & PARTNER
## PATENTANWÄLTE - EUROPEAN PATENT ATTORNEYS

Dr. Nicolaus ter Meer, Dipl.-Chem.
Frithjof E. Müller, Dipl.-Ing.
Mauerkircherstrasse 45
D-8000 MÜNCHEN 80

Helmut Steinmeister, Dipl.-Ing.
Manfred Wiebusch
Artur-Ladebeck-Strasse 51
D-4800 BIELEFELD 1

Case: Y-2543-2                29.6.1987

**Yuko YONEDA**

1-16 Akebono-cho,

Toyama-shi

Toyama-ken

Japan

---

**Method of detecting specific protein**

---

Priorität:    June 30, 1986, Japan, No. 61-153707

## FIELD OF THE INVENTION

The present invention relates to a simple and rapid method of detecting very small amounts of a specific protein in fluids using with the immunological reaction, and to the reagents and a set of equipments to make the detection.

The domain applicable to this detecting method includes the kits for the detection of various specific proteins in the fundamental research, various clinical diagnostic agents, and the kits for the detection of various diseases in agriculture, stock raising and marine products industry, and the like.

BACKGROUND

For the purpose of detecting specific proteins (or peptides), the fundamental research and the clinical applications such as diagnosis of various diseases etc. are principally mentioned.

Among these, in the fundamental research, for example, in the domains of biochemistry and immunology, it goes without saying that the detection of specific proteins is an indispensable means. Moreover, in the area of clinical diagnosis, the detection of specific proteins has become an essential factor. Namely, in certain diseases, the proteins specific to the diseases (for example, hormone, enzyme, etc.) are always released into the body fluids. In these cases, not only the diseases can be diagnosable through the detection of specific proteins thereof, but also the lapse of diseases can be known through the detection followed up with the lapse of time.

As presumed from the significance aforementioned, the detection of specific protein should have high reliability. For example, in order to diagnose the diseases unmistakably, high accuracy capable of detecting specific protein specifically is required.

Next, high sensitivity is necessary. For example, in the diagnosis of diseases, it is desirable, of course, to have correct diagnosis as early as possible. For this reason, it is necessary to be able to detect even extremely small amounts of specific protein.

Further, the facts that the procedure for detection is simple and can be used at any time and anywhere, that the result can be obtained rapidly and that the preservation and the transportation of the results are also indispensable factors.

The method of detecting specific protein which has been used most widely hitherto is that used the immunological antigen-antibody reaction. This is the method wherein the antibody which recognizes the specific protein to become antigen is prepared based on the property that the antibody recognizes the antigenic substance specifically, this is allowed to react with specific protein in the test sample through antigen-antibody reaction, and the result is detected using secondary phenomena such as, for example, coagulation reaction etc. and labels.

Such detecting methods are classified roughly further into the qualitative detecting method and the quantitative detecting method according to the degree of detection. That is, the qualitative detecting method is one to detect the existence of more than a fixed amount of protein by simple means such as tinting and is applied generally to clinical diagnostic agents etc.

Whereas, the quantitative detecting method is a system

- 3 -

to determine the specific protein quantitatively and has higher sensitivity and accuracy compared with the qualitative detecting method, so that it is used widely for fundamental research and clinical diagnosis.

In following, the representative examples which are the methods of detecting specific proteins having been used most widely hitherto and are applied to the clinical diagnostic agents which are mentioned below.

(1)  A method applied the coagulation reaction of erythrocytes.

(2)  A method applied the coagulation reaction with latex.

(3)  Radioimmunoassay method (RIA method)

(4)  Enzyme immunoassay method (EIA method)

Among these, with the methods due to the coagulation reaction, that is, (1) the method applied the coagulation reaction of erythrocytes and (2) the method applied the coagulation reaction with latex, special equipments and high-grade measurement apparatus are unnecessary and the detection is performed simply, but there have been numerous miscalculation because of low sensitivity and ambiguous judgment. Recently, in these methods, the improvements such as that the antibody being altered to a monoclonal antibody having high specificity and the like have been made to raise the sensitivity, but there still remains a point incapable of overcoming, as it is estimated from the fundamental principle, since the result of the antigen-antibody

- 4 -

reaction is to be identified through the indirect phenomenon.

On the contrary, between immunoassay methods under (3) and (4), the radioimmunoassay method, which was developed by S.A. Berson and R. Yallow in 1959, has become established today as an indispensable one. The principle of the detecting method is to quantify the antigen-antibody reaction in whatever form and to directly determine the antigen or antibody. Here, the method of labeling the antigen or antibody with something as a means therefor is called labeling immunoassay method and the quantification is made on the basis of this label. Since the radioimmunoassay method uses a radioactive isotope with high sensitivity as a label, it can detect quantitatively even extremely small amounts of protein antigen. Therefore, it has contributed significantly not only to the fundamental research but also to the clinical diagnosis.

In order to carry out the radio immunoassay method, however, the radioactive substances have to be used. Consequently, special facilities are needed and extreme caution must be exercised in handling this method. Moreover, there is also the problem of disposing the radioactive wastes from this method because society is against the use of radioactive materials. Thus it is hard to say that this method will be useful in the future.

Therefore, the method attracting the largest attention in recent is the enzyme immunoassay method under (4). This is a labeling immunoassay method using enzymes as the label.

There are many kinds of determining methods for this, among which the most practical sandwich method will be described.

Preparing two kinds of antibodies specific to an antigen, one is allowed to bond to the solid phase and the other is bonded with enzyme to become a label to obtain a complex. First, the test sample solution is added to the solid phase bonded with antibody and unreacted substances are removed by washing sufficiently. Then, if the enzyme-antibody complex is allowed to react, a bonded matter in "sandwich" form composed of the antibody-antigenic protein complex is produced on the surface of the solid phase. Secondly, after the excess complexes completely removed, the enzymic activity at the complex bonded on the surface at the solid phase is determined to quantify the specific antigenic protein in the test sample.

A problem in this detecting method is the need of completely removing the unreacted enzyme-antibody complex. This is because an enzyme with high specific activity is used in order to raise the sensibility, therefore if there is even a slight amounts of this complex remaining, the background of the measurement is raised. The shortcoming of using the enzyme as a label cannot be avoided and the difficulty in the procedure removing the unreacted agents cannot be eliminated. Accordingly, several simple qualitative methods which applied the sandwich method such as enzyme immunoassay methods are devised, but all of them have the same problem described above and there remains a problematic point that perfect accuracy cannot be obtained.

However, the detecting method required most urgently at present is one in which special equipment and apparatus are unnecessary and highly accurate and reliable data can be obtained rapidly by a simple procedure. For instance, when clinical diagnosis is urgently needed after an operation, etc., it is essential to make judgment accurately and still rapidly for the sake of preparing subsequent treatment. It is natural that high accuracy and rapidity are sought on the results obtained by the diagnostic agent, which becomes the material for the judgment.

Therefore, in order to satisfy these requirements, a method capable of detecting very small amounts of specific protein simply and rapidly with high sensitivity and high accuracy, by the conventional detecting methods, has been invented. The development of the invention has taken a great amounts of diligent efforts.

SUMMARY OF THE INVENTION

The invention relates to a method of detecting specific protein characterized in that, by the use of monoclonal antibodies or a combination of a polyclonal antibody and a kind of a monoclonal antibody having high specificity. The specific protein which is included in the test sample is prefiltered for removing the obstractive residue, and is inserted in a sandwich form between the antibodies, and detected visually as a tinted spot produced directly on the test piece of paper with the detecting agent
(metallic colloidal solution bonded with another monoclonal
antibody).

BRIEF DESCRIPTION OF THE DRAWINGS

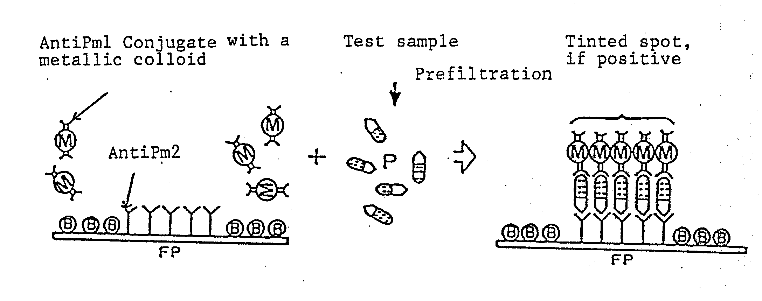

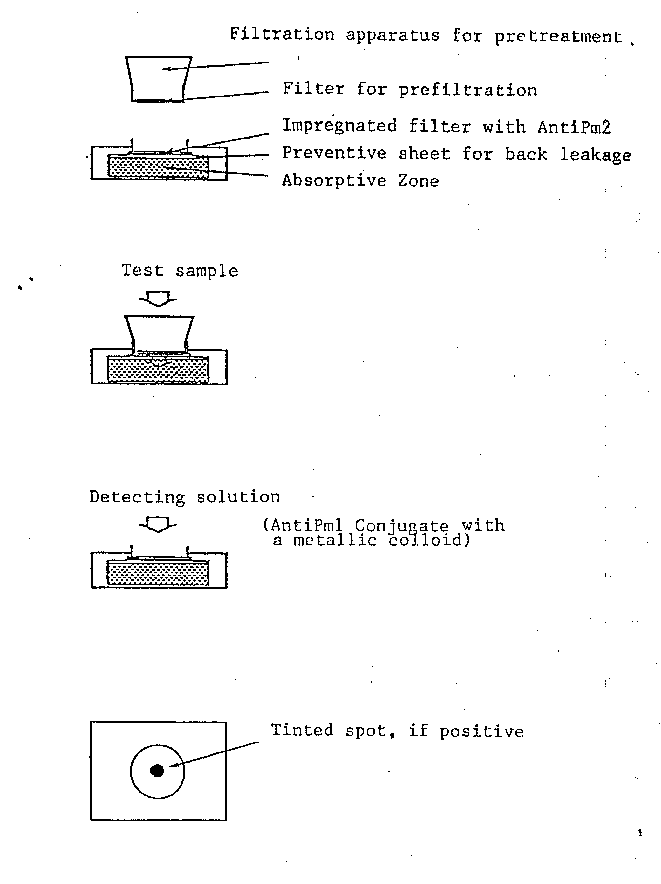

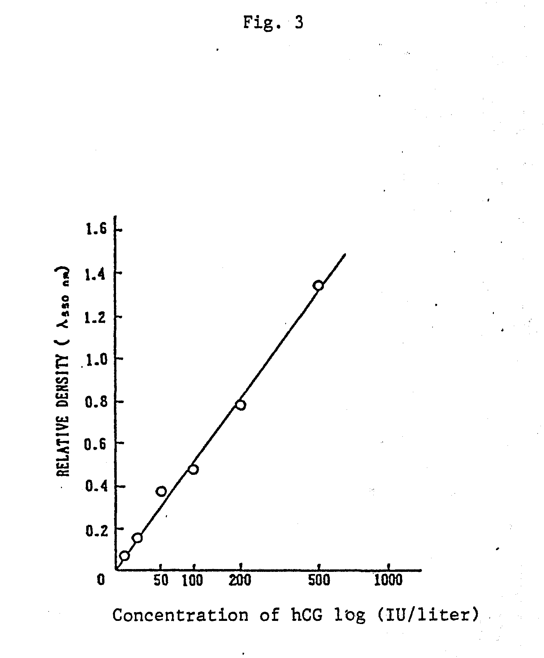

Fig. 1 is diagram illustrated showing one example of the
fundamental principle of the invention. Fig. 2 is a diagram
illustrating an example (2 - 2) of the invention. Fig. 3 is
a diagram showing clearly the performance of the invention.

DETAILED DESCRIPTION OF THE INVENTION

As the monoclonal antibody is defined as "a pure antibody
against only one antigen determinant" (Dictionary of Biology,
3rd edition, Iwanami Bookstore), the specificity thereof against
antigen is exceptionally high compared with the usual antibody
(called polyclonal antibody to monoclonal antibody). For this
reason, utilizing this property, an improvement is made in raising
the accuracy by using this monoclonal antibody in the method
of detecting specific protein applied to the conventional antigen-
antibody reaction.

However, in the first place, it cannot be necessarily said
that the epitope is specific for only a certain protein. Some
are common to a plurality of different proteins. For this reason,
the specific detection is said to be difficult for some kinds
of proteins. In order to overcome this, the retrieval of monoclonal
antibodies is vigorously conducted against specific antigen
determinants consuming a lot of time and labor (for example,
Japanese Unexamined Patent Publication No. Sho 60-236069).

According to the invention, a method has been developed which makes detectable even extremely small amounts of specific protein detectable, using monoclonal antibodies against a plurality of different epitopes without daring to use expensive monoclonal antibodies having such high specificity.  This will be explained below:

As a method to specifically recognize particular protein, in the invention, the detection accuracy has been enhanced and the specificity has been raised by double antigen-antibody reactions. Moreover, the direct  detecting method of labeling the immunoassay method was applied and, as a detecting substance, colloidal metal particles (metallic colloidal particles) were used.  These metallic colloidal particles are tinted with a unique color due to their respective physical properties and therefore, the result of the antigen-antibody reaction can be directly and visually displayed.  The colloidal metal particles are known in the field of immunochemistry research.

As a representative example of these colloidal metal particles, colloidal gold particles (hereafter referred to as colloidal gold) are widely used.  These are ones having made from aurichloric acid colloidal under particular conditions and are tinted from pink to reddish purple depending on the diameter of the colloidal gold particle.  when this colloidal gold and the protein are placed under certain conditions, they are bonded through noncovalent and electrostatic adsorption to form a stable conjugate.  Since this is a bonding not necessitated by chemical modification, there is no denaturation of the protein nor in the physiological activity reduced. In the world of immunohistology, colloidal gold is already known as an immunostainer for microscopic research, and has been used for many years. The metallic colloidal particles represented by this colloidal gold have been adopted as a detecting substance for specific protein.

The method of detecting specific protein utilizing this metallic colloid was already reported as the sol particle immunoassay method (SPIA method) (Japanese Unexamined Patent Publication No. Sho 55-15100). Also, the clinical diagnostic agent uses colloidal gold among other metallic colloids (trade name "Predictor Color D") is already on the market. The principle thereof lies in the fact that the test sample is added to the solution of gold colloid-antibody conjugate and the coagulation of the conjugate taking place through the immunosedimentation reaction between antigenic substance in the sample and antibody is judged by the accompanying fading of the solution of colloidal gold (from red to colorless). With this detecting method, the sensitivity is low (more than 400 international units of hCG in 1 liter of urine) and the detection time of required is long (about 30 minutes). In particular, when the amount at detecting substance is very small, there remains a problem also in the point of accuracy because the fading is so indistince, it is difficult to make an accurate judgment. Moreover after a lapse of time, the fading occurs even when it is negative. In addition, the result of the judgment cannot be preserved. In order to overcome such

- 10 -

problems, the fading of the conjugate of the metallic colloid in the state of solution was not untilized, but a method capable of clear and accurate judgment by anyone and still capable of the preservation of the result thereof has been devised. Moreover, in order to enhance the detecting sensitivity and accuracy, a method of removing the obstructive substances which become the origin of the high background at the time of detection has also been devised together.

In Fig. 1, the fundamental principle of the invention is shown. In this case, two monoclonal antibodies against a plurality of mutually different epitopes are used. Here, P indicates the specific protein or peptide to be detected, and the monoclonal antibody against "epitope 1" on this P molecule is expressed by AntiPml and that against "epitope 2" is expressed by AntiPm2.

AntiPml forms the conjugate with metallic colloid (M) and is made up of the detecting solution after the $_pH$ buffer solution is added. On the other hand, AntiPm2 is impregnated into an absorptive carrier (FP) and, after air-drying, the carrier is dipped into a buffer solution containing the blocking agent (B, eg. bovine serum albumin etc.) to mask the surface of FP except for the areas where AntiPm2 has been bonded thereto for the purpose of the preventing bonding with nonspecific protein.

At the time of detection, the specific protein P in a fixed quantity of the solution of test sample is caught with AntiPm2 on FP. When allowed to react thereto with the conjugate, the specific protein P is recognized by both monoclonal antibodies of AntiPml and AntiPm2 and, as shown in the diagram, the complex composed of AntiPm2-specific protein-AntiPml-conjugate with colloidal gold is produced on the absorptive carrier FP through the antigen-antibody reaction. The result is that only the complex bonded on the surface of FP remains as a tinted spot to be identified.

When using the polyclonal antibody in combination with the monoclonal antibody having high specificity, the former may be regarded as AntiPm2 and the latter as AntiPml.

This can be applied to a simple clinical diagnostic agent etc. as a method to detect more than a fixed amount of specific protein qualitatively. Moreover the spot obtained can be determined quantitatively with densitometer (Fig. 3).

Furthermore, if provided ones varies stepwise the amount of AntiPml used impregnate the absorptive carrier (FP), the specific protein can be also detected semiquantitatively similarly to the so-called dot blotting method used most frequently in the domain of fundamental biochemistry without the necessity of analytical instruments.

Next, taking into consideration the factors that obstruct the results in this detecting method, if the metallic colloid detecting agent is bonded nonspecifically on to the absorptive carrier FP, the background upon detection becomes relatively high. But, as to this, the nonspecific bonding of conjugate of metallic colloid on to the surface of test paper was hindered by masking the areas other than those the antibody on the surface of FP being absorbed thereto with skim milk, bovine serum albumin (BSA), etc.

Moreover, when the test samples are original urine, etc., unknown substances stay on the detecting test paper to catch the conjugate of metallic colloid nonspecifically on the test paper, so that the phenomenon to render the background high upon detection tends to occur, but, in the invention, a filtration apparatus for the pretreatment of the test sample has been devised as a means to overcome this. The filtration apparatus is compared of a nylon membrane filter coated with a solution of skim milk, $pH$ buffer solution, etc. (refer to Fig. 2). By using this coating treatment, it is possible to prevent the protein in the test sample from being caught nonspecifically on the filter during the prefiltration stage. By this prefiltration, nonspecific unknown obstructive substances in the test sample can be removed and $pH$ can be kept as optimum as possible. As a result, both the deteacting sensitivity and the detecting accuracy are improved drastically (refer to Table 1).

Further, in order to make the conjugate of metallic colloid stable in the reaction solution, $pH$ buffer solution, stabilizers, etc. were added to the conjugate solution of metallic colloid.

Next, in regard to the possibility that other protein exists in the solution of the test sample that has the same epitope as epitope 2 on the specific protein P which is being detected, P2 reacts to bond with AntiPm2 on the carrier, but, unless epitope 1 against AntiPml is existent on P, the conjugate does not bond and therefore no mistakes are made in the detection. A problem here is the case when epitope 1 is also existent on P2, but, at this time, the problem is solved by obtaining the monoclonal antibody against the other epitope. However, the probability of the existance of protein holding two kinds of different epitopes in common exists in generally considered to be rather low. Moreover, when combining polyclonal antibodies with monoclonal antibodies, the problem can be solved by making the specificity of the monoclonal antibody to produce the conjugate with metallic colloid as high as possible.

As can be understood from the facts above, the specific protein detectable by this detecting method should have two or more kinds of epitopes or the epitope specific to the antigen thereof. In general, many proteins have subunits structure. Accordingly, in the case of protein consisting of a plurality of subunits, only a plurality of monoclonal antibodies specific to the epitopes on the surface of the protein of respective subunits has only to be obtained and, even in the case of protein or peptide not having a subunit structure, it can be detected specifically by the present detecting method, even with the existence of a plurality of different epitopes.

As described above, the invention provides a simple and rapid method of detecting the specific protein, wherein the specificity thereof has been enhanced and the accuracy has been raised by the recognition of specific protein using a plurality of monoclonal antibodies or a combination of a monoclonal antibody having high specificity and a polyclonal antibody, and, at the same time, the sensitivity has been increased by using stable metallic colloid as a detecting agent so as to be detected visually.

EXAMPLE

As an example of the invention, detection of human chorionic gonadotropin (hCG) is mentioned, hCG is a hormone secreted specifically at the early days of pregnancy or in the cases of certain kinds of chorionic and nonchorionic tumors. At present, various methods to detect hCG released into urine with anti-hCG antibodies have been devised and are used widely as the indicator not only for the diagnosis of pregnancy but also for the diagnosis and therapy of tumors aforementioned.

However, hCG is glycoprotein hormone consisting of both $\alpha$-and $\beta$-subunits. Since the $\alpha$-subunit therebetween has a structure that very closely resembles the respective subunits of hLH (human luteinizing hormone), hFSH (human follicle-stimulating hormone) and hTSH (human thyroid-stimulating hormone), the crossing property with conventional polyclonal antibodies is high and, in particular, the possibility of having a false positive reaction cannot be eliminated. Particularly, when detecting extremely low concentrations of hCG as in the case of prognosis at the time of abortion, and the diagnosis of extrauterine pregnancy, etc., the conven-

- 15 -

tional polyclonal antibodies could not be used at all. There, in particular, since the arranged portion of amino acid from C end to 28th or 30th of the $\beta$- subunit of hCG is unique, efforts are made to prepare the monoclonal antibody which makes against this portion epitope and therefore lessen the crossing property with hLH etc. to enhance the accuracy. But, for this , a high degree of technology is necessary and a lot of labor and time should be consumed.

For this reason, in this invention, efforts were diligently made to make a highly accurate hCG detecting system without daring to obtain such expensive anti-$\beta$-hCG monoclonal antibodies. Moreover, since the system to determine extremely small amounts of hCG strictly and quantitatively has already been established with RIA or EIA, a simple procedure of qualitative detecting method has been devised as in the following examples.

(1)  Preparation of monoclonal antibodies

The spleen obtained from mice immunized with commercial hCG was allowed to fuse with myeloma cells by the common method to obtain hybridoma.  This was submitted to cloning and, using $\alpha$ -and $\beta$-subunits as standard substances, the specificity of the monoclonal antibody was assayed for each clone by the ELISA method.

When the clone producing specific antibodies against the respective $\alpha$-and $\beta$-subunit was further restrictively diluted and submitted to subcloning, the production of specific antibodies was observed in 25% of the clone in the $\alpha$-subunit and 40% of

clone in the $\beta$-subunit. These were proliferated and injected into the abdominal cavity of mice in the usual way to obtain ascites,and monoclonal antibodies were purified through affinity column.

(2) Example of making kit for the detection of hCG and procedure thereof

(2 - 1) Method of paper matrix test

Among the monoclonal antibodies obtained in (1) above, anti-$\alpha$-hCG monoclonal antibody was impregnated into the filter. After air-drying, this was dipped into the solutions of BSA etc. to block it and then air dried to fix it to the supporter. On the other hand, anti-$\beta$-hCG antibody was bonded to the commercial solution of gold colloid as usual.

The procedure is as follows: The above mentioned filter aforementioned is dipped for a fixed period of time (for example, 1 minute) into the urine which has been pretreated by the filtration apparatus, to catch hCG in the urine by anti-$\alpha$-hCG monoclonal antibodies on the filter. Then, this filter is placed in the solution of conjugate of gold colloid and allowed to stand for some time (about 10 minutes). Thereafter, the filter is taken out and, after rinsing slightly, it is dried in air.

If it is possitive, a reddish purple spot will come out at the center of filter. If nagative, any background cannot be seen.

(2 - 2) Method with filter

Two components are obtained similarly to (2 - 1 ). Namely, the filter impregnated with the anti-α-hCG monoclonal antibody and the gold colloidal solution conjugated with the anti-β-hCG monoclonal antibody are provided. The former is placed on the top surface of the cylindrical vessel and the lower section is filled up with a highly hygroscopic material (refer to Fig. 2).

A sample of the urine to be tested is taken into an injection tube, this is submitted to prefiltration with the filtration apparatus, and a fixed amount of this is added drop by drop onto the center of the test paper of the kit to catch the hCG on the filter. Following this, the solution of conjugate of colloidal gold is added drop by drop. If it is possitive, a reddish purple spot is observed immediately at the center.

(3)  Performance

As a result of the investigation to detect the sensitivity of this kit using hCG as a standard substance, hCG of a concentration of as low as 5 IU/liter could be detected. The sensitivity is over 100 times greater than that of the conventional simplified detecting method. Moreover, when making the kit, as in the example in (2-2), the specific protein is immediately identified as a spot after the addition of conjugate of colloidal gold. At this time, depending on the test samples, the background upon detection happens to become high. But, by using the filtration apparatus for pretreatment of the invention, the background, at the time of detection, disappears completely to make it possible to drastically enhance the detecting sensitivity and the detecting accuracy for a specific protein. Moreover, the necessary time for this method is less than two minutes, which shows the unparalleled rapidity. Furthermore, the result obtained remains - 18 - on the test paper which makes it possible to transport it by
the way of the post office etc.  It is also possible to preserve
it as a permanent record.  These features have not been possible
with the various detecting kits that are commonly used at
present. Needless to say, the accuracy of detection is an indispensable
factor especially in the case of clinical diagnosis.

Moreover, as shown in Table 1, the detection is not hindered
to any degree in very high concectration of one hundred million
IU/liter and, because of the general absence of false negative
called "prozone phenomenon", so the test sample can be used
without dilution.

Table 1 Test of detecting sensitivity of hCG

| Concn. of hCG (IU/liter) | 0 | 2 | 5 | 10 | 20 | 50 | 100 | 500 | $1 \times 10^9$ |
|---|---|---|---|---|---|---|---|---|---|
| Judgment | | − | ± | + | + | ++ | ++ | ++ | ++ | +++ |

The invention comprises the following:
(1)  A method of detecting specific protein (or peptide) characterized in that, upon the recognition and detection of protein
(or peptide) aiming at the detection in the test sample which
has the obstructive substances removed with the filtration
apparatus by the combination of monoclonal antibodies or a polyclonal
antibody with a kind of monoclonal antibody having high specificity against a plurality of mutually different epitopes (antigen
determinants).  The detection and identification are made by
allowing the protein to trap on the test piece of paper  fixed with one monoclonal antibody or more, or one polyclonal antibody.
Then tinting rapidly takes place on the test piece of paper
by using metallic colloidal particles with other monoclonal
antibodies (monoclonal antibody having high specificity in the
case of using polyclonal antibody on one hand) bonded with colloidal
metal particles as a detecting agent. The kit includes the necessary
apparatus for the test.

(2)   A test piece of paper for the detection of specific protein
(or peptide) in the test sample characterized in that, in the
detecting method according to claim 1, a buffer solution containing one of monoclonal antibody or more, or one polyclonal antibody
against the protein or peptide which is being detected is impregnated
into the nylon membrane filter as an absorbtive carrier and,
after air drying, the areas except where the antibody is bonded
are blocked using blocking agents such as skim milk etc.

(3)   A reagent for the detection of specific protein (or peptide)
in the test sample characterized in that, in the detecting method
according to claim 1, the colloidal metal particles bonded with
another monoclonal antibodies against same antigen, which is
different from the monoclonal antibody used in claim 2 (monoclonal
antibody having high specificity in the case of having used
polyclonal antibody), are used as detecting solutions as they
are, or they are made powdered by freeze-drying together with
buffer solution and used after dissolved at the time of use.

(4)   A set of instruments used for the kit, wherein, in the detecting method according to claim 1, the test piece according to claim 2 and the reagents according to claim 3 are combined to make a kit that detects simply and rapidly specific protein (or peptide) in the test sample.

(5)  A filtration apparatus for the pretreatment of the test sample mainly composed of a filter coated with skim milk etc., which is used for the pretreatment of the test sample.  The purpose is to enhance the detecting accuracy of the apparatus used for the method of detecting specific protein or peptide according to claim 1.

## Claims

1. A method for detecting a specific protein (or peptide) **characterized by** reacting a test sample containing said protein (or peptide), from which obstructive substances have been removed by filtration, with one or more monoclonal antibodies or polyclonal antibodies specific for said protein (or peptide) and detecting the result of the antigen-antibody reaction with a detecting agent comprising a conjugate of colloidal metal particles and of different monoclonal antibodies having a high specificity against a plurality of mutually different epitopes (antigen determinants) of said protein (or peptide).

2. The method according to claim 1, **characterized by** contacting the test sample with an absorptive carrier to which the monoclonal antibodies or polyclonal antibodies specific for said protein (or peptide) are fixed and thereafter treating the absorptive carrier with a solution of said detecting agent.

3. The method according to claim 1 or 2, **characterized by** prefiltering the test sample with a filter coated with skim milk.

4. A reagent for carrying out the method according to claims 1 to 3, **characterized by** an absorptive carrier impregnated with a buffer solution containing said one or more monoclonal antibodies or polyclonal antibodies specific for said protein (or peptide), dried and treated with a blocking agent, such as skim milk, on the areas not covered with said antibodies.

5. The reagent according to claim 4 **characterized in that** the absorptive carrier is a test piece of paper or a nylon membrane filter.

6. A detecting agent for carrying out the method according to claims 1 to 3, **characterized by** comprising colloidal particles bonded with monoclonal antibodies having a high specificity against a plurality of mutually different epitopes (antigen determinants) of said protein (or peptide) and different from those present on the absorptive carrier as defined in claim 4.

7. The detecting agent according to claim 6 **characterized by** comprising a solution or the freeze-dried product, which if desired contains a buffer.

8. A reagent kit comprising the reagent according to claims 4 and 5 and the detecting agent according to claims 6 and 7.

9. A filtration apparatus for the pretreatment of the test sample used in the method according to claims 1 to 3, **characterized by** a filter coated with skim milk etc.

Fig. 1

AntiPml Conjugate with a metallic colloid — Test sample — Tinted spot, if positive

Prefiltration

AntiPm2

FP

P

FP

Fig. 2

Filtration apparatus for pretreatment

Filter for prefiltration

Impregnated filter with AntiPm2
Preventive sheet for back leakage
Absorptive Zone

Test sample

Detecting solution

(AntiPml Conjugate with
a metallic colloid)

Tinted spot, if positive

Fig. 3

Concentration of hCG log (IU/liter)